# EUROPEAN PATENT SPECIFICATION

(11) **EP 3 362 429 B1**
(45) Date of publication and mention of the grant of the patent: **07.12.2022**
(21) Application number: 16779097.1
(22) Date of filing: 11.10.2016
(51) Int. Cl.: B01J 10/00, C07C 51/363, C07C 51/43, C07C 51/44

(54) **PROCESS FOR THE PREPARATION OF MONOCHLOROACETIC ACID**
VERFAHREN ZUR HERSTELLUNG VON MONOCHLORESSIGSÄURE
PROCESSUS DE PRÉPARATION D'ACIDE MONOCHLOROACÉTIQUE

(30) Priority: 13.10.2015 EP 15189637
(43) Date of publication of application: 22.08.2018
(73) Proprietor: Nouryon Chemicals International B.V., 6824BM Arnhem (NL)
(72) Inventor: VOS, Hendrik Jan, 7313 GK Apeldoorn (NL); TOLLIN, Lars Magnus, 663 31 Skoghall (SE); KOOIJMAN, Cornelis, 7412 AC Deventer (NL)
(74) Representative: LKGlobal UK Ltd.
(86) International application number: PCT/EP2016/074256
(87) International publication number: WO 2017/064015

(56) References cited:
- WO-A1-2013/057125
- GB-A- 727 074

## Description

The present invention pertains to a process for the preparation of monochloroacetic acid and an apparatus suitable for use in this process.

Chloroacetic acids can be obtained by reacting acetic acid with chlorine in a chlorination step, resulting in the formation of a chloroacetic acid stream and HCI as side product. Acetic anhydride is often added as catalyst. The resulting chloroacetic acid stream generally comprises a mixture of the desired product monochloroacetic acid (MCA) with "overchlorinated" products like dichloroacetic acid (DCA) and trichloroacetic acid (TCA). In particular, DCA may be present in the reaction product mixture in an amount of up to 6 wt%. To convert these "overchlorinated" products to monochloroacetic acid, the product of the chlorination reaction is often subjected to a hydrogenation step, in which the dichloroacetic acid and other overchlorinated compounds are converted to monochloroacetic acid. In the prior art this hydrogenation step is sometimes also referred to as dechlorination step. This results in the formation of a product stream comprising monochloroacetic acid and a further side product stream comprising HCl.

However, it was found that when subjecting the resulting chloroacetic acid stream to a dechlorination step, the presence of acid chlorides and/or acid anhydrides may interfere with the dechlorination step, creating a dark-colored product due to the formation of aldehydes, which give rise to the formation of condensation products. Further, excessive formation of aldehydes may cause fouling in the dechlorination reactor and downstream equipment. It also adds to the emission to the environment of the production site. Furthermore, it is desirable to reduce the use of acetic anhydride and acetyl chloride, which is used as a catalyst for the chlorination step.

Therefore, prior to feeding the chloroacetic stream to the dechlorination step, a purification step has to be conducted wherein the acid chlorides and anhydrides, such as acid chlorides and anhydrides of monochloroacetic acid and acetic acid, are removed from the liquid chloroacetic acid stream.

CN104058947 describes a method for the production of monochloroacetic acid wherein the chloroacetic acid stream is subjected to distillation, followed by catalytic hydrogenation. In the distillation all the HCI leaves at the top. The bottom of the distillation does not contain water and HCl. Therefore, acid anhydrides are formed in the bottom of the distillation. The presence of these acid anhydrides interferes with the dechlorination step, creating a dark-colored product due to the formation of aldehydes, which give rise to the formation of condensation products. Further, excessive formation of aldehydes may cause fouling in the dechlorination reactor and downstream equipment. It also adds to the emission to the environment of the production site. Furthermore, these formed anhydrides are not recovered.

CN104649887 also describes a process for the production of monochloroacetic acid wherein the chloroacetic acid stream is subjected to vacuum distillation followed by a three stage condensation. This process has the same drawbacks as the process of CN104058947.

In WO 2013/057125 a process is disclosed for the purification of a liquid feed comprising monochloroacetic acid and dichloroacetic acid wherein a small amount of water is added to the liquid feed prior to the dechlorination. The liquid feed is subjected to a stripping operation using HCI gas to remove anhydrides and/or acid chlorides in the feed, prior to subjecting it to the water addition and subsequent dechlorination.

In GB 928179 the hot off-gas from the chlorination reactor is fed to an absorber. The HCI gas from the chlorination is fed to a three stage absorption process. The first step comprises cooling the gas, the second step comprises a washing step with the acetic acid feed and a third step comprises a saturation of the acetic acid feed with HCl. This document describes a batch process.

In NL 109769 the HCI gas leaving the chlorination is fed directly to a stripper, where an atmospheric stripping process is conducted at 110°C. The HCI gas leaving the stripper is led directly to an absorber.

In US 4,281,184 a continuous chlorination process is described, with a stripping and an absorption section. The HCI gas from the chlorination is fed to the acetic acid absorber after a single step condensation step. The HCI gas from this acetic acid absorber is cooled to 15°C and fed to the stripper. After the stripper the HCI gas is also cooled to below 35°C and fed to the acetyl chloride absorber.

GB 727,074 is directed to the production of monochloroacetic acid, wherein gaseous chlorine is passed in to a mixture of glacial acetic acid and acetyl chloride or acetic anhydride. In order to inhibit formation of dichloroactic acid an ionizing agent is added to the reaction mixture. The acetyl chloride formed is distilled off using hydrogen chloride as a sweep at a temperature of between 80 and 120°C. Subsequently the acid chloride is recovered by absorption in glacial acetic acid. The residue of the reaction, containing ionizing agent and MCA, is subjected to fractional distillation. However, GB 727,074 does not disclose any cooling step between the stripper and the absorbing section.

There is need in the art for a process for the production of monochloroacetic acid which allows efficient dechlorination and which process is attractive from a technical, environmental and economic point of view. The present invention provides such a method.

The present invention pertains to a process for the chlorination of acetic acid to form monochloroacetic acid comprising the following steps
a. reacting acetic acid with chlorine using acetic anhydride and/or acetyl chloride as catalyst to form a liquid phase and a gaseous phase,
   - the liquid phase comprising at least monochloroacetic acid, acid chlorides, acid anhydrides and optionally unreacted acetic acid, and
   - the gaseous phase comprising HCI and acetic acid, monochloroacetic acid, acid anhydrides and acid chlorides,
b. stripping said liquid phase at a temperature of between 120 and 180°C and a pressure between 1 and 7 bara with gaseous HCI to form a gaseous HCI stream comprising acetic acid, acid chlorides and optionally acid anhydrides and monochloroacetic acid, and a liquid stream comprising monochloroacetic acid and dichloroacetic acid;
c. cooling the gaseous HCI stream comprising acetic acid, acid chlorides and optionally acid anhydrides and monochloroacetic acid to a temperature of between 10 and 60°C (preferably around 35°C), and
d. feeding the cooled gaseous HCI to an absorber with acetic acid as absorbent absorbing the acetic acid, acid chlorides and optionally monochloroacetic acid and acid anhydrides, thus forming a stream comprising acetic acid and acid chlorides.

It was found that with the process according to the invention a monochloroacetic acid stream is formed comprising a very low amount of anhydrides. Furthermore, the acid chlorides are removed more effectively from the liquid phase than with other known methods such as distillation or with the method according to GB 727,074. Furthermore, the process according to the invention can be operated continuously.

In a preferred embodiment of the process the acetic acid and acid chlorides-comprising stream obtained in step d is re-directed to step a. Thus, with this process the catalyst is removed both from the liquid phase and the gaseous phase of the chlorination reaction and re-directed into the process. When redirecting the acetic acid and acid chlorides to the chlorination process a very economical consumption of catalyst is obtained.

It is noted that the term "acid chlorides" as used throughout the specification denotes acetyl chloride or a mixture thereof with chloroacetyl chloride and/or dichloroacetyl chloride. The term "(acid) anhydrides" as used throughout the specification denotes acetic acid anhydride, optionally mixed with one or more anhydrides selected from the group consisting of acetic acid anhydride, DCA anhydride, MCA anhydride, DCA-MCA anhydride, acetic acid - MCA anhydride and acetic acid - DCA anhydride.

In the process according to the invention the chlorination process of step a is preferably conducted in a chlorination reactor. Chlorination reactors are known in the art and need no further elucidation here.

Besides HCI, the gaseous phase of the chlorination comprises acetic acid, monochloroacetic acid, acid chlorides and acid anhydrides. Typically, the amounts of the components of the gaseous phase comprise, besides HCI, 5-15 wt% acetic acid, 1-10 wt% monochloroacetic acid, 5-20 wt% acetyl chloride, 5-15 wt% chloroacetyl chloride and 0.1-0.2 wt% acid anhydrides.

In a preferred embodiment the gaseous phase from step a comprising HCI and acetic acid, monochloroacetic acid, acid chlorides and acid anhydrides is subjected to a condensation step, wherein at least part of the acetic acid, acid chlorides, acid anhydrides and monochloroacetic acid is removed from the HCI gas, resulting in purified HCI gas. Said condensation step may be a single condensation step, but also multi-condensation steps may be used. The condensation may take place in one or more heat exchangers. The acid chlorides, acetic acid, monochloroacetic acid and acid anhydrides will condense upon lowering of the temperature and may be re-directed to the chlorination step. This also contributes to the overall yield of the entire process. The HCI gas is at the same time purified by the removal of the acid chlorides, acetic acid, monochloroacetic acid and acid anhydrides and may be used in the stripping step. In the purified HCI gas less than 10 wt%, preferably less than 2 wt% or even less than 1 wt% of acetyl chloride is present and less than 0.5 wt%, preferably less than 0.1 wt%, in some embodiments less than 0.05 wt% of acetic acid, monochloroacetic acid, chloroacetyl chloride and acid anhydrides is present.

The stripping of the liquid phase is conducted by guiding HCI gas through the liquid phase. The acid chlorides, acid anhydrides and optionally unreacted acetic acid present in the liquid phase are removed from the liquid phase by the HCI gas. In fact, the concentration of acid anhydrides is lowered *per se* by the presence of the hydrogen chloride, because of the change of equilibrium. Said stripping step may be accomplished by simply bubbling the HCI gas through the liquid product stream, but preferably the product stream is fed through a gas stripper.

Stripping is a physical separation process where one or more components are removed from a liquid stream by a vapor stream. In industrial applications the liquid and vapor streams can have co-current or countercurrent flows. Stripping is usually carried out in either a packed or a trayed column.

Stripping is mainly conducted in trayed towers (plate columns) and packed columns, collectively referred to as stripping towers, and less often in spray towers, bubble columns and centrifugal contactors.

Trayed towers consist of a vertical column with liquid flowing in at the top and out at the bottom. The vapor phase enters in the bottom of the column and exits out of the top. Inside the column there are trays or plates. These trays force the liquid to flow back and forth horizontally while the vapor bubbles up through holes in the trays. The purpose of these trays is to increase the amount of contact area between the liquid and vapor phases.

Packed columns are similar to trayed columns in that the liquid and vapor flows enter and exit in the same manner. The difference is that in packed towers there are no trays. Instead, packing is used to increase the contact area between the liquid and vapor phases. Many different types of packing are used and each one has advantages and disadvantages.

As mentioned previously, stripping towers can be trayed or packed. Packed columns, and particularly when random packing is used, are usually favored for smaller columns with a diameter less than 0.6 m and a packed height of not more than 6 m. Packed columns can also be advantageous for corrosive fluids, high foaming fluids, when fluid velocity is high, and when particularly low pressure drop is desired. Trayed strippers are advantageous because of ease of design and scale up. Structured packing can be used similar to trays despite possibly being the same material as dumped (random) packing. Using structured packing is a common method to increase the capacity for separation or to replace damaged trays.

Trayed strippers can have sieve, valve, or bubble cap trays while packed strippers can have either structured packing or random packing. Trays and packing are used to increase the contact area over which mass transfer can occur as mass transfer theory dictates. Packing can have varying material, surface area, flow area, and associated pressure drop. Older generation packing include ceramic Raschig rings and Berl saddles. More common packing materials are plastic Pall rings and ceramic Intalox saddles. Each packing material of this newer generation improves the surface area, the flow area and/or the associated pressure drop across the packing. Also important is the ability of the packing material to not stack on top of itself. If such stacking occurs, it drastically reduces the surface area of the material.

Conventional stripping towers may be used in the process according to the invention. They are known in the art and need no further elucidation here.

In conventional processes where a stripping step is applied in order to remove acid chlorides, such a stripping step is typically conducted at temperatures of between 80 and 120°C, at atmospheric pressure. The temperature range of between 80 and 120°C is a logical choice for a skilled person as the boiling point of acetyl chloride is 52°C.

However, we have found that it has an advantage to conduct the stripping at a temperature of between 120 and 180°C and a pressure between 1 and 7 bara so as to form a gaseous HCI stream comprising acid chlorides and optionally acetic acid. By conducting the stripping step at a temperature of at least 120 °C, preferably, at least 122°C, more preferably at least 125°C, and most preferably at least 130°C, it is ensured that any chloroacetyl chloride, if present, is also removed from the liquid phase.

Preferably, the stripping step is conducted at a temperature of at most 180°C, more preferably 170°C, and most preferably 160°C. The temperature required for the stripping step may be obtained by heating the HCI with steam. In a preferred embodiment, in step b, the liquid phase is stripped at a pressure between 1 and 7 bara with gaseous HCI having a temperature of between 120 and 180°C to form a gaseous HCI stream comprising acetic acid, acid chlorides and optionally acid anhydrides and monochloroacetic acid, and a liquid stream comprising monochloroacetic acid and dichloroacetic acid. It is also possible to recycle the bottom product stream over a steam heater in order to obtain the required temperature in the stripping step. In a further preferred embodiment, the liquid stream formed in the stripper comprising monochloroacetic acid and dichloroacetic acid may be fed directly to the dechlorination step.

In a preferred embodiment the stripping step is performed at a pressure of at least 1.6 bara, preferably at least 3 bara, more preferably at least 4 bara and most preferably at least 5 bara. Preferably, the stripping step is performed at a pressure not higher than 10 bara, more preferably not higher than 8 bara. The advantage of performing this stripping step under elevated pressure is that due to the higher pressure in the stripper column, more HCI will dissolve in the bottom product of the stripper column, thus preventing the formation of anhydrides from acid chlorides. As a result, the liquid stream formed in the stripper comprising monochloroacetic acid and dichloroacetic acid comprises less anhydride or even no anhydride at all. This is an advantage because, as already described, said liquid stream is now suitable for being fed directly to the dechlorination step.

Subsequently, the gaseous stream coming from the stripper is cooled to a temperature of between 10 and 60°C, preferably around 35°C, and fed to an absorber using acetic acid as the absorbent. In the absorber, acid chlorides are absorbed in the acetic acid. The HCI gas leaves the absorber at the top and may be fed to a HCI purification and absorption section.

For the absorption step an absorption tower may be used. Absorption towers are also packed or plate columns as described above and are known in the art. Absorption towers and need no further elucidation here.

At least part of the liquid stream leaving the bottom of the absorber, which comprises acid chlorides and chloroacetic acid, is preferably fed to the chlorination step. The optimal removal of this catalyst from both the liquid phase and the gaseous phase of the chlorination reactor creates a process with a very economical consumption of catalyst, a high yield of monochloroacetic acid and a monochloroacetic stream stripped of components that may interfere with the dechlorination step.

The present invention is further directed to an apparatus which is suitable for the process according to the invention. The apparatus according to the invention comprises:
- a chlorination reactor with at least two inlets, an upper outlet and a lower outlet,
- a stripping tower with an upper inlet and a lower inlet, an upper outlet and a lower outlet, which upper inlet is directly or indirectly connected with the lower outlet of the chlorination reactor,
- at least one condenser with an upper inlet, a lower outlet for gas and a lower outlet for liquid, which lower outlet for liquid is connected with the upper inlet of the chlorination reactor and which lower outlet for gas is directly or indirectly connected with the lower inlet of the stripping tower,
- a cooler with an upper inlet and a lower outlet
- an absorption tower with a lower inlet and an upper outlet, an upper inlet and lower outlet, which lower inlet is directly or indirectly connected with the upper outlet of the stripping tower and which lower outlet is directly or indirectly connected with the inlet of the chlorination reactor

wherein the condenser and absorption tower are disposed above the chlorination reactor and the stripping tower is located below the chlorination reactor or at approximately the same height as the chlorination reactor, and wherein the upper outlet of the stripping tower is connected to the upper inlet of the cooler, and the lower outlet of the cooler is connected to the lower inlet of the absorption tower.

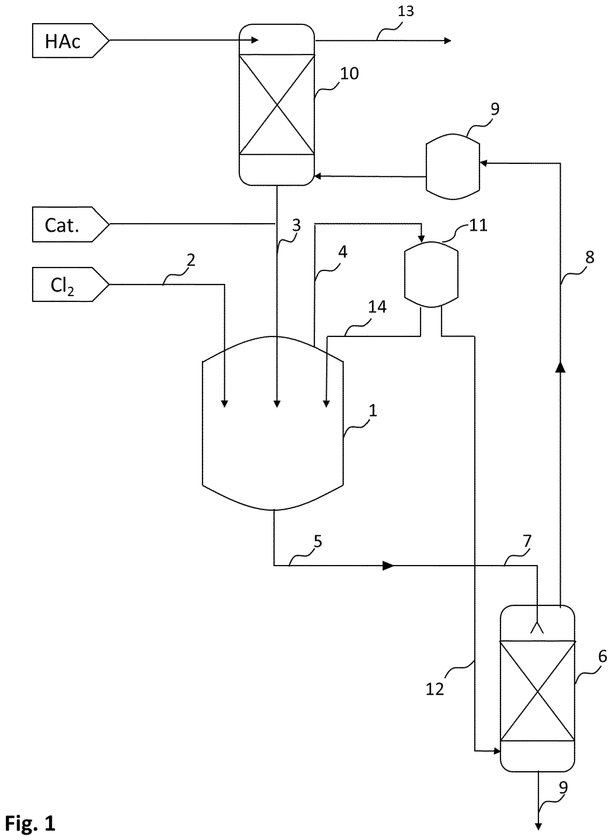

The present invention will be further elucidated by Figure 1, which gives a schematic view of an apparatus which is used to perform one of the embodiments of the process according to the invention.

### Figure 1

This figure gives a schematic view of an apparatus which is used in the process according to the invention. To a chlorination reactor (1) with an inlet for chlorine (2) and an inlet for the catalyst and acetic acid (3), the reactants chlorine, acetic acid and acetyl chloride and/or acetic anhydride are led. The resulting gaseous stream comprising HCI, acetic acid, acid chlorides and acid anhydrides and optionally monochloroacetic acid is led via an upper outlet (4) to a condenser (11). In the condenser (11) acetic acid, acid chlorides, acid anhydrides and any monochloroacetic acid are condensed and recycled via outlet 14 to the chlorination reactor (1). The purified HCI is led to the lower inlet (12) of the stripper (6).

The resulting liquid stream comprising monochloroacetic acid, dichloroacetic acid, acid chlorides, acid anhydrides and optionally unreacted acetic acid is led via a lower outlet (5) to the upper inlet (7) of a stripping tower (6) which is fed with HCI gas via lower inlet (12). In the stripping tower (6) a liquid stream is formed which comprises monochloroacetic acid, dichloroacetic acid. Said liquid stream is led via outlet 9 for further processing in the dechlorination unit. The resulting liquid stream comprises less than 0.1 wt% acid anhydrides and less than 0.1 wt% acid chlorides, based on total liquid stream. By carefully controlling the pressure in the stripper the presence of any acid anhydride in the resulting liquid stream can be avoided altogether. For instance, at a temperature of 155°C and a pressure of 3.2 bara, no acid anhydride was present in the resulting liquid stream.

The gaseous stream formed in the stripper comprises hydrogen chloride, acid chlorides and traces of acid anhydrides. Said gaseous stream is led to a cooler (9) via the upper outlet (8) of the stripper (6). In the cooler (9) the gaseous stream is cooled to a temperature between 10 and 60°C (preferably around 35°C). Said cooled stream is fed to an absorber (10) with an acetic acid inlet and a HCI outlet (13), wherein the acid chlorides and acetic anhydrides are absorbed in acetic acid and combinedly fed to the chlorination reactor. The recovery rate of the catalyst is thus more than 99 wt% in the process according to the invention.

As is clear from the schematic view, the condenser and absorption tower are disposed above the chlorination reactor and the stripping tower is located below the chlorination reactor, so as to make optimal use of the effect of gravity on the flow of materials, reducing the complexity of the plant.

## Claims

1. Process for the chlorination of acetic acid to form monochloroacetic acid comprising the following steps
a. reacting acetic acid with chlorine using acetic anhydride and/or acetyl chloride as catalyst to form a liquid phase and a gaseous phase,
- the liquid phase comprising at least monochloroacetic acid, acid chlorides, acid anhydrides and optionally unreacted acetic acid, and
- the gaseous phase comprising HCI and acetic acid, acid chlorides, acid anhydrides and monochloroacetic acid,
b. stripping said liquid phase at a temperature of between 120 and 180°C and a pressure between 1 and 7 bara with gaseous HCI to form a gaseous HCI stream comprising acetic acid, acid chlorides and optionally acid anhydrides and monochloroacetic acid, and a liquid stream comprising monochloroacetic acid and dichloroacetic acid;
c. cooling the gaseous HCI stream comprising acetic acid, acid chlorides and optionally acid anhydrides and monochloroacetic acid to a temperature of between 10 and 60°C, and
d. feeding the cooled gaseous HCI to an absorber with acetic acid as absorbent absorbing the acetic acid, acid chlorides and optionally monochloroacetic acid forming a stream comprising acetic acid and acid chlorides.

2. Process according to claim 1 wherein the temperature of step b is between 120 and 160°C.

3. Process according to claim 1 wherein the acetic acid and acid chlorides-comprising stream obtained in step d is re-directed to step a.

4. Process according to any one of the preceding claims wherein the gaseous HCI which is used in step b is heated with steam prior to being used to strip the liquid phase.

5. Process according to any one of the preceding claims wherein step a is conducted in a chlorination reactor.

6. Process according to any one of the preceding claims wherein step b is conducted in a stripping tower.

7. Process according to any one of the preceding claims wherein the gaseous phase comprising HCI, acetic acid, acid chlorides, acid anhydrides and monochloroacetic acid is subjected to a condensation step, wherein at least part of the acid chlorides, acid anhydrides, acetic acid and monochloroacetic acid is removed from the HCI gas, resulting in purified HCI gas.

8. Process according to claim 7, wherein the condensation step takes place in a condenser.

9. Process according to claim 7, wherein the purified HCI gas retrieved is used in step b, the stripping step, of claim 1.

10. Process according to any one of the preceding claims 7-8 wherein the acid chloride, acetic acid, acid anhydride and monochloroacetic acid removed from the HCI gas are recycled to step a, the chlorination step of claim 1.

11. Process according to any one of the preceding claims wherein the process is conducted continuously.

12. Process according to any one of the preceding claims, wherein the said liquid stream of step b) comprising monochloroacetic acid and dichloroacetic acid is directly fed to a dechlorination step.

13. Apparatus for the chlorination of acetic acid to form monochloroacetic acid comprising:
- a chlorination reactor with at least two inlets, an upper outlet and a lower outlet,
- a stripping tower with an upper inlet and a lower inlet, an upper outlet and a lower outlet, which upper inlet is directly or indirectly connected with the lower outlet of the chlorination reactor,
- at least one condenser with an upper inlet, a lower outlet for gas and a lower outlet for liquid, which lower outlet for liquid is connected with the upper inlet of the chlorination reactor and which lower outlet for gas is directly or indirectly connected with the lower inlet of the stripping tower,
- a cooler with an upper inlet and a lower outlet,
- an absorption tower with a lower inlet and an upper outlet, an upper inlet and lower outlet, which lower inlet is directly or indirectly connected with the upper outlet of the stripping tower and which lower outlet is directly or indirectly connected with the inlet of the chlorination reactor,
wherein the condenser and absorption tower are disposed above the chlorination reactor and the stripping tower is located below the chlorination reactor or at approximately the same height as the chlorination reactor, and
wherein the upper outlet of the stripping tower is connected to the upper inlet of the cooler, and the lower outlet of the cooler is connected to the lower inlet of the absorption tower.

## Patentansprüche

1. Verfahren zur Chlorierung von Essigsäure, um Monochloressigsäure zu bilden, umfassend die folgenden Schritte
a. Reagieren von Essigsäure mit Chlor unter Anwendung von Essigsäureanhydrid und/oder Acetylchlorid als Katalysator, um eine flüssige Phase und eine gasförmige Phase zu bilden,
- wobei die flüssige Phase mindestens Monochloressigsäure, Säurechloride, Säureanhydride und wahlweise unreagierte Essigsäure umfasst und
- wobei die gasförmige Phase HCl und Essigsäure, Säurechloride, Säureanhydride und Monochloressigsäure umfasst,
b. Abscheiden der flüssigen Phase bei einer Temperatur zwischen 120 und 180 °C und einem Druck zwischen 1 und 7 bara mit gasförmigem HCl, um einen gasförmigen HCl-Strom, der Essigsäure, Säurechloride und wahlweise Säureanhydride und Monochloressigsäure umfasst, und einen flüssigen Strom, der Monochloressigsäure und Dichloressigsäure umfasst, zu bilden;
c. Kühlen des gasförmigen HCl-Stroms, der Essigsäure, Säurechloride und wahlweise Säureanhydride und Monochloressigsäure umfasst, auf eine Temperatur zwischen 10 und 60 °C und
d. Führen des gekühlten gasförmigen HCl zu einem Absorber mit Essigsäure als Absorptionsmittel, das die Essigsäure, Säurechloride und wahlweise Monochloressigsäure unter Bildung eines Stroms absorbiert, der Essigsäure und Säurechloride umfasst.

2. Verfahren nach Anspruch 1, wobei die Temperatur von Schritt b zwischen 120 und 160 °C liegt.

3. Verfahren nach Anspruch 1, wobei der Essigsäure und Säurechloride umfassende Strom, der in Schritt d erhalten worden ist, zu Schritt a umgelenkt wird.

4. Verfahren nach einem der vorhergehenden Ansprüche, wobei der gasförmige HCl, der in Schritt b verwendet wird, mit Dampf erhitzt wird, bevor er zum Abscheiden der flüssigen Phase verwendet wird.

5. Verfahren nach einem der vorhergehenden Ansprüche, wobei der Schritt a in einem Chlorierungsreaktor ausgeführt wird.

6. Verfahren nach einem der vorhergehenden Ansprüche, wobei der Schritt b in einem Abscheideturm ausgeführt wird.

7. Verfahren nach einem der vorhergehenden Ansprüche, wobei die gasförmige Phase, die HCl, Essigsäure, Säurechloride, Säureanhydride und Monochloressigsäure umfasst, einem Kondensationsschritt unterworfen wird, wobei mindestens ein Teil der Säurechloride, Säureanhydride, Essigsäure und Monochloressigsäure aus dem HCl-Gas entfernt wird, was zu gereinigtem HCl-Gas führt.

8. Verfahren nach Anspruch 7, wobei der Kondensationsschritt in einem Kondensator stattfindet.

9. Verfahren nach Anspruch 7, wobei das gereinigte, zurückgewonnene HCl-Gas in Schritt b, dem Abscheideschritt nach Anspruch 1, verwendet wird.

10. Verfahren nach einem der vorhergehenden Ansprüche 7-8,
wobei das Säurechlorid, die Essigsäure, das Säureanhydrid und die Monochloressigsäure, die aus dem HCl-Gas entfernt worden sind, zu Schritt a, dem Chlorierungsschritt nach Anspruch 1, rückgeführt werden.

11. Verfahren nach einem der vorhergehenden Ansprüche, wobei das Verfahren kontinuierlich ausgeführt wird.

12. Verfahren nach einem der vorhergehenden Ansprüche, wobei der flüssige Strom aus Schritt b), der Monochloressigsäure und Dichloressigsäure umfasst, direkt zu einem Entchlorungsschritt geführt wird.

13. Vorrichtung für die Chlorierung von Essigsäure, um Monochloressigsäure zu bilden, umfassend:
- einen Chlorierungsreaktor mit mindestens zwei Einlässen, einem oberen Auslass und einem unteren Auslass,
- einen Abscheideturm mit einem oberen Einlass und einem unteren Einlass, einem oberen Auslass und einem unteren Auslass, welcher obere Einlass direkt oder indirekt mit dem unteren Auslass des Chlorierungsreaktors verbunden ist,
- mindestens einen Kondensator mit einem oberen Einlass, einem unteren Auslass für Gas und einem unteren Auslass für Flüssigkeit, welcher untere Auslass für Flüssigkeit mit dem oberen Einlass des Chlorierungsreaktors verbunden ist und welcher untere Auslass für Gas direkt oder indirekt mit dem unteren Einlass für den Abscheideturm verbunden ist,
- eine Kühlvorrichtung mit einem oberen Einlass und einem unteren Einlass,
- einen Absorptionsturm mit einem unteren Einlass und einem oberen Auslass, einem oberen Einlass und einem unteren Auslass, welcher untere Einlass direkt oder indirekt mit dem oberen Auslass des Abscheideturms verbunden ist und welcher untere Auslass direkt oder indirekt mit dem Einlass des Chlorierungsreaktors verbunden ist,
wobei der Kondensator und der Absorptionsturm über dem Chlorierungsreaktor angeordnet sind und der Abscheideturm sich unter dem Chlorierungsreaktor oder in etwa derselben Höhe wie der Chlorierungsreaktor befindet und
wobei der obere Auslass des Abscheideturms mit dem oberen Einlass der Kühlvorrichtung verbunden ist und der untere Auslass der Kühlvorrichtung mit dem unteren Einlass des Absorptionsturms verbunden ist.

## Revendications

1. Procédé de chloration d'acide acétique pour former de l'acide monochloroacétique comprenant les étapes suivantes
a. mise en réaction de l'acide acétique avec du chlore en utilisant de l'anhydride acétique et/ou du chlorure d'acétyle comme catalyseur pour former une phase liquide et une phase gazeuse,
- la phase liquide comprenant au moins de l'acide monochloroacétique, des chlorures d'acide, des anhydrides d'acide et éventuellement de l'acide acétique n'ayant pas réagi, et
- la phase gazeuse comprenant de l'HCl et de l'acide acétique, des chlorures d'acide, des anhydrides d'acide et de l'acide monochloroacétique,
b. extraction de ladite phase liquide à une température comprise entre 120 et 180 °C et une pression comprise entre 1 et 7 bara avec de l'HCl gazeux pour former un flux d'HCl gazeux comprenant de l'acide acétique, des chlorures d'acide et éventuellement des anhydrides d'acides et de l'acide monochloroacétique, et un flux liquide comprenant de l'acide monochloroacétique et de l'acide dichloroacétique ;
c. refroidissement du flux d'HCl gazeux comprenant de l'acide acétique, des chlorures d'acide et éventuellement des anhydrides d'acide et de l'acide monochloroacétique à une température comprise entre 10 et 60 °C, et
d. alimentation de l'HCl gazeux refroidi dans un absorbeur avec de l'acide acétique comme absorbant qui absorbe l'acide acétique, les chlorures d'acide et éventuellement l'acide monochloroacétique formant un flux comprenant de l'acide acétique et des chlorures d'acide.

2. Procédé selon la revendication 1 dans lequel la température de l'étape b est comprise entre 120 et 160 °C.

3. Procédé selon la revendication 1 dans lequel le flux comprenant de l'acide acétique et des chlorures d'acide obtenu à l'étape d est redirigé vers l'étape a.

4. Procédé selon l'une quelconque des revendications précédentes dans lequel l'HCl gazeux qui est utilisé à l'étape b est chauffé avec de la vapeur avant d'être utilisé pour extraire la phase liquide.

5. Procédé selon l'une quelconque des revendications précédentes dans lequel l'étape a est réalisée dans un réacteur de chloration.

6. Procédé selon l'une quelconque des revendications précédentes dans lequel l'étape b est réalisée dans une tour d'extraction.

7. Procédé selon l'une quelconque des revendications précédentes dans lequel la phase gazeuse comprenant de l'HCl, de l'acide acétique, des chlorures d'acide, des anhydrides d'acide et de l'acide monochloroacétique est soumise à une étape de condensation, dans lequel au moins une partie des chlorures d'acide, des anhydrides d'acide, de l'acide acétique et de l'acide monochloroacétique est éliminée de l'HCl gazeux, résultant en un HCl gazeux purifié.

8. Procédé selon la revendication 7, dans lequel l'étape de condensation a lieu dans un condenseur.

9. Procédé selon la revendication 7, dans lequel l'HCl gazeux purifié récupéré est utilisé à l'étape b, l'étape d'extraction, de la revendication 1.

10. Procédé selon l'une quelconque des revendications 7 à 8 précédentes dans lequel le chlorure d'acide, l'acide acétique, l'anhydride d'acide et l'acide monochloroacétique éliminés de l'HCl gazeux sont recyclés à l'étape a, l'étape de chloration de la revendication 1.

11. Procédé selon l'une quelconque des revendications précédentes dans lequel le procédé est réalisé de manière continue.

12. Procédé selon l'une quelconque des revendications précédentes, dans lequel ledit flux liquide de l'étape b) comprenant de l'acide monochloroacétique et de l'acide dichloroacétique est directement alimenté à une étape de déchloration.

13. Appareil de chloration de l'acide acétique pour former de l'acide monochloroacétique comprenant :
- un réacteur de chloration avec au moins deux entrées, une sortie supérieure et une sortie inférieure,
- une tour d'extraction avec une entrée supérieure et une entrée inférieure, une sortie supérieure et une sortie inférieure, laquelle entrée supérieure est directement ou indirectement reliée à la sortie inférieure du réacteur de chloration,
- au moins un condenseur avec une entrée supérieure, une sortie inférieure pour le gaz et une sortie inférieure pour le liquide, laquelle sortie inférieure pour le liquide est reliée à l'entrée supérieure du réacteur de chloration et laquelle sortie inférieure pour le gaz est directement ou indirectement reliée à l'entrée inférieure de la tour d'extraction,
- un refroidisseur avec une entrée supérieure et une sortie inférieure,
- une tour d'absorption avec une entrée inférieure et une sortie supérieure, une entrée supérieure et une sortie inférieure, laquelle entrée inférieure est directement ou indirectement reliée à la sortie supérieure de la tour d'extraction et laquelle sortie inférieure est directement ou indirectement reliée à l'entrée du réacteur de chloration,
dans lequel le condenseur et la tour d'absorption sont agencés au-dessus du réacteur de chloration et la tour d'extraction est située au-dessous du réacteur de chloration ou approximativement à la même hauteur que le réacteur de chloration,
et
dans lequel la sortie supérieure de la tour d'extraction est reliée à l'entrée supérieure du refroidisseur, et la sortie inférieure du refroidisseur est reliée à l'entrée inférieure de la tour d'absorption.
